# EUROPEAN PATENT SPECIFICATION

(11) **EP 4 387 510 B1**
(45) Date of publication and mention of the grant of the patent: **20.05.2026**
(21) Application number: 22782605.4
(22) Date of filing: 18.08.2022
(51) Int. Cl.: A61B 5/00, A61B 5/06, A61B 6/12, A61B 8/12

(54) **SYSTEMS AND METHODS OF IDENTIFYING VESSEL ATTRIBUTES USING EXTRAVASCULAR IMAGES**
SYSTEME UND VERFAHREN ZUR IDENTIFIZIERUNG VON GEFÄSSATTRIBUTEN UNTER VERWENDUNG EXTRAVASKULÄRER BILDER
SYSTÈMES ET PROCÉDÉS D'IDENTIFICATION D'ATTRIBUTS DE VAISSEAUX À L'AIDE D'IMAGES EXTRAVASCULAIRES

(30) Priority: 19.08.2021 US 202163234916 P
(43) Date of publication of application: 26.06.2024
(73) Proprietor: Lightlab Imaging, Inc., Westford, MA 01886 (US)
(72) Inventor: CHIU, Wei, Stony Brook, NY 11790 (US); LI, Shimin, Acton, MA 01720 (US); GOPINATH, Ajay, Bedford, MA 01730 (US); SEGEV, Jacob, Haifa (IL); DASCAL, Lorina, 3499701 Haifa (IL)
(74) Representative: Boult Wade Tennant LLP
(86) International application number: PCT/US2022/040755
(87) International publication number: WO 2023/023248

(56) References cited:
- WO-A1-2020/077381
- WO-A2-2022/126101
- VAN KRANENBURG MATTHIJS ET AL: "Validation of Renal Artery Dimensions Measured by Magnetic Resonance Angiography in Patients Referred for Renal Sympathetic Denervation", ACADEMIC RADIOLOGY, ELSEVIER, AMSTERDAM, NL, vol. 22, no. 9, 7 July 2015 (2015-07-07), pages 1106 - 1114, XP029255205, ISSN: 1076-6332, DOI: 10.1016/J.ACRA.2015.03.014
- "36th Annual Meeting of the Japanese Society of Neuroradiology, 8-10 February 2007, Kagawa, Japan", NEURORADIOLOGY ; A JOURNAL DEVOTED TO NEUROIMAGING AND INTERVENTIONAL NEURORADIOLOGY OFFICIAL JOURNAL OF THE EUROPEAN SOCIETY OF NEURORADIOLOGY ORGAN OF THE JAPANESE NEURORADIOLOGICAL SOCIETY, SPRINGER, BERLIN, DE, vol. 49, no. 7, 26 June 2007 (2007-06-26), pages 587 - 618, XP019513094, ISSN: 1432-1920, DOI: 10.1007/S00234-007-0254-9

## Description

### CROSS-REFERENCE TO RELATED APPLICATION

The present application claims the benefit of the filing date of U.S. Provisional Patent Application No. 63/234,916 filed August 19, 2021, entitled Systems and Methods of Identifying Vessel Attributes Using Extravascular Images.

### BACKGROUND OF THE INVENTION

Calculation of a blood vessel's Virtual Flow Reserve ("VFR") can be used to diagnose lesions within the blood vessel and can assist in determining regions in which therapeutic measures, such as placement of stent, should occur. VFR computation can be computed using intravascular images. However, intravascular imaging will often only occur over a portion of the entire blood vessel. For example, some intravascular optical coherence tomography ("OCT") systems may have a maximum pullback length of 75 mm, while the entire length of the blood vessel may be more than twice that length. Without additional information regarding the structure of the blood vessel at the regions that have not been imaged by an intravascular probe, assumptions are made in order to perform VFR computations. In particular, assumptions are made regarding the structure of the vessel in the region of the vessel that has not been intravascularly imaged. However, to the extent the structure of the vessel differs from these assumptions, the VFR computation may include inaccuracies. Systems and methods are needed to reduce these inaccuracies of VFR computations and thereby improve diagnosis of blood vessel conditions.

van Kranenburg et al., Acad. Radiol. 2015; 22:1106-1114 presents validations using intravascular ultrasound of some renal artery dimensions measured by magnetic resonance angiography. Sakamoto et al., abstract O-02 in Neuroradiology (2007) 49:587-618 reports on comparisons of MR angiography source images with IVUS data of cartoid plaque fibrous caps. WO2020/0773891 describes a process for evaluating a coronary region using optical coherence tomography or intravascular ultrasound and angiogram images. WO2022/126101, which forms part of the prior art under Article 54(3) EPC, provides arrangements for determining location of a medical device in a body lumen using imaging markers and a location sensor on a flexible elongate instrument, and displaying a composite image comprising a correlated diagnostic scan and the imaging markers.

### BRIEF SUMMARY OF THE INVENTION

The invention provides a computer implemented method for identifying attributes of a blood vessel as set out in claim 1, and a system as set out in claim 12. Systems and methods are disclosed for identifying features of a blood vessel using extravascular and intravascular images in order to estimate a virtual flow reserve (VFR) of the imaged blood vessel. Aspects of the disclosure include using extravascular images to estimate the size of the blood vessel in regions that have not been intravascularly imaged. The VFR estimation may be based on a resistance model that incorporates both the intravascular image data and the estimated blood vessel size. In other aspects, multiangled extravascular images are captured and analyzed in order to identify the size and orientation of branch vessels.

The systems and methods may include one or more memories for storing images of a vessel and one or more processors configured to: capture a plurality of extravascular images of a vessel during a pullback of an intravascular imaging probe having a defined pullback length along a first region of the vessel; detect locations of one or more markers in the plurality of extravascular images; correlate the first region of the vessel represented in the plurality of extravascular images with the pullback length based on the location of the one or more markers in the plurality of extravascular images; and determine a size of a second region of the vessel represented in the plurality of extravascular images based the correlation of the first region of the vessel represented in the plurality of extravascular images with the pullback length.

In other aspects of the disclosure, the size of the second region of the vessel may include at least one of a length of the second region, a cross-section diameter within the second region, and a cross-sectional area within the second region. In still other aspects, the one or more processors may be further configured to compute a virtual flow reserve (VFR) of the vessel based on a plurality of images captured by the intravascular imaging probe and based on the determined size of the second region of the vessel. In addition, the VFR of the vessel may be computed based, at least in part, on a distance between a vessel centerline and a boundary of the vessel the second region of the vessel identified in at least one of the plurality of extravascular images.

In still other aspects of the disclosure, the second region of the vessel may include at least one of a distal epicardial region and a proximal epicardial region of the vessel. In addition, correlating the first region of the vessel represented in the plurality of extravascular images with the pullback length may include scaling a lumen size represented in at least one of the plurality of the extravascular images.

In yet other aspects, the one or more processors may be further configured to analyze the plurality of extravascular images so as to identify a position and takeoff angle of a branch relative to the vessel. In addition, the one or more processors may be further configured to compute a virtual flow reserve (VFR) of the vessel based on the identified position and takeoff angle of the branch.

In other aspects of the disclosure, the one or more processors may be configured to receive a plurality of extravascular images captured at a plurality of angulations relative to a patient; generate a three-dimensional model of a vessel based on the plurality of extravascular images; identify locations within the vessel for which an intravascular pullback procedure will be conducted; estimate a size of the vessel at one or more regions not included within the identified locations, wherein the estimation is based on the three-dimensional model; and compute a virtual flow reserve (VFR) of the vessel based on the estimated size of the vessel.

In still other aspects of the disclosure, the one or more processors may be further configured to identify one or more bifurcations along the vessel, and estimate a size and orientation of a branch vessel at the one or more bifurcations, wherein computing the VFR of the vessel is based on the estimated size and orientation of the branch vessel, and wherein the orientation of the branch vessel comprises a takeoff angle of the branch vessel relative to the vessel.

### BRIEF DESCRIPTION OF THE DRAWINGS

Fig. 1 is a schematic diagram of a system configured to image blood vessels, automatically detecting features/regions of interest relative to image data obtained, including computation and display of data relating to the virtual flow reserve within regions of the blood vessels.
Fig. 2 is a diagram representing a blood vessel in accordance with aspects of the disclosure.
Fig. 3 is a diagram of a resistance model generated in accordance with aspects of the disclosure.
Fig. 4 is a diagram representing positions of intravascular probe markers in accordance with aspects of the disclosure.
Fig. 5 is a diagram representing a position of an intravascular probe within a vessel in accordance with aspects of the disclosure.
Figs. 6-8 are displays of angiographic images of a vessel containing an intravascular probe in accordance with aspects of the disclosure.
Fig. 9 is a display of multiangled angiographic images in accordance with aspects of the disclosure.
Fig. 10 is a diagram of a vessel bifurcation and vessel centerlines in accordance with aspects of disclosure.
Figs. 11-12 are flowcharts for analyzing extravascular images in accordance with aspects of the disclosure.

### DETAILED DESCRIPTION

Systems and methods of the current disclosure provide for improved assessment of blood vessel attributes, including an improved computation of the virtual flow reserve ("VFR") within one or more blood vessels. In calculating the VFR of the vessel based on the OCT images, may perform feature detection and alignment of relative imaging datasets from an intravascular imaging pullback. For example, the intravascular imaging pullback may be an OCT, intravascular ultrasound ("IVUS"), or near-infrared spectroscopy pullback. The imaging data sets may be taken at one or more points in time corresponding to different arterial events or treatments. One or more representations of an artery may be displayed based on the imaging data set. The representations may include an indication of identification of VFR. The one or more representations may be displayed to a user.

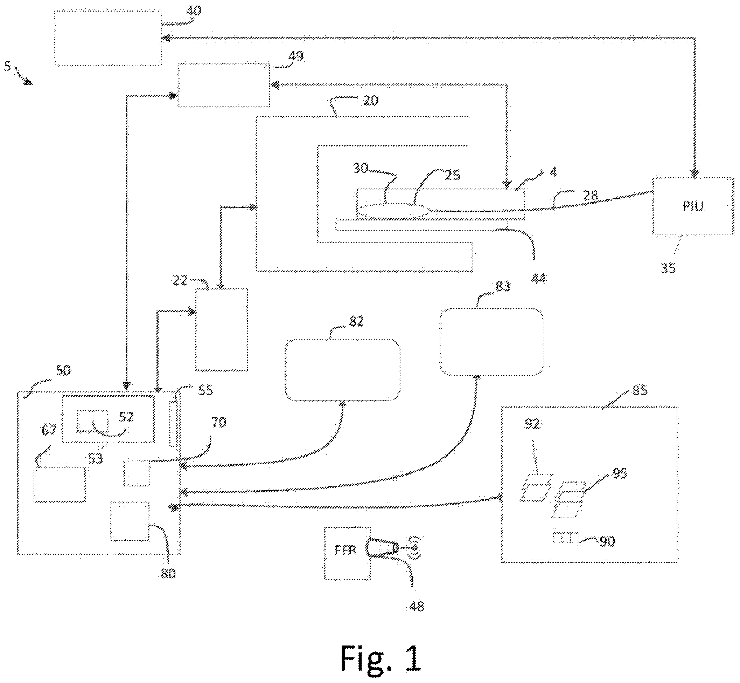

FIG. 1 is a schematic diagram of a diagnostic system 5 suitable for imaging arteries and other blood vessels, and is configured to automatically identifying blood vessel attributes of interest based on the image data that is obtained. The system 5 is suitable for viewing and assess a visual representation of arterial information. These user interfaces can include one or more moveable elements that can be controlled by a user with a mouse, joystick, or other control and can be operated using one or more processors and memory storage elements. Morphology results automatically obtained relative to image data can be displayed as part of a streamlined workflow.

FIG. 1 shows a system 5 which includes various data collection subsystems suitable for collecting data or detecting a feature of or sensing a condition of or otherwise diagnosing a subject 4. In one embodiment, the subject is disposed upon a suitable support 44 such as table bed to chair or other suitable support. Typically, the subject 4 is the human or another animal having a particular region of interest 25.

The data collection system 5 includes a noninvasive imaging system such as a nuclear magnetic resonance, x-ray, computer aided tomography, or other suitable noninvasive imaging technology. As shown as a non-limiting example of such a noninvasive imaging system, an angiography system 20 such as suitable for generating cines is shown. The angiography system 20 can include a fluoroscopy system. Angiography system 20 is configured to noninvasively image the subject 4 such that frames of angiographic data, in the form of frames of image data. The angiographic data may be generated independently or in connection with a pullback procedure that is performed using a probe 30 such that a blood vessel in region 25 of subject 4 is imaged using one or more intravascular imaging technologies, such as OCT, IVUS, or near-infrared spectroscopy, as well as the noninvasive angiographic imaging.

The angiography system 20 is in communication with an angiography data storage and image management system 22, which can be implemented as a workstation or server in one embodiment. In one embodiment, the data processing relating to the collected angiography signal is performed directly on the detector of the angiography system 20. The images from system 20 are stored and managed by the angiography data storage and image management 22.

In one embodiment system server 50 or workstation 85 handle the functions of system 22. In one embodiment, the entire system 20 generates electromagnetic radiation, such as x-rays. The system 20 also receives such radiation after passing through the subject 4. In turn, the data processing system 22 uses the signals from the angiography system 20 to image one or more regions of the subject 4 including region 25.

As shown in this particular example, the region of interest 25 is a subset of the vascular or peripherally vascular system such as a particular blood vessel. This can be imaged using OCT. A catheter-based data collection probe 30 is introduced into the subject 4 and is disposed in the lumen of the particular blood vessel, such as for example, a coronary artery. The probe 30 can be a variety of types of data collection probes such as for example an OCT probe, an FFR probe, an IVUS probe, a near-infrared spectroscopy probe, a probe combining features of two or more of the foregoing, and other probes suitable for imaging within a blood vessel. The probe 30 may include a probe tip, one or more radiopaque markers, an optical fiber, and a torque wire. Additionally, the probe tip may include one or more data collecting subsystems such as an optical beam director, an acoustic beam director, a pressure detector sensor, other transducers or detectors, and combinations of the foregoing.

For a probe that includes an optical beam director, the optical fiber 28 is in optical communication with the probe with the beam director. The torque wire defines a bore in which an optical fiber is disposed. In FIG. 1, the optical fiber 28 is shown without a torque wire surrounding it. In addition, the probe 30 also includes the sheath such as a polymer sheath (not shown) which forms part of a catheter. The optical fiber 28, which in the context of an OCT system is a portion of the sample arm of an interferometer, is optically coupled to a patient interface unit (PIU) 35 as shown.

The patient interface unit 35 may include a probe connector suitable to receive an end of the probe 30 and be optically coupled thereto. The data collection probes 30 may be disposable. The PIU 35 includes suitable joints and elements based on the type of data collection probe being used. For example, a combination OCT and IVUS data collection probe will be operated with an OCT and IVUS PIU. The PIU 35 may include a motor suitable for pulling back the torque wire, sheath, and optical fiber 28 disposed therein as part of the pullback procedure. In addition to being pulled back, the probe tip may be rotated by the PIU 35. In this way, a blood vessel of the subject 4 can be imaged longitudinally or via cross-sections.

In turn, the PIU 35 is connected to one or more intravascular data collection systems 40. The intravascular data collection system 40 can be an OCT system, an IVUS system, another imaging system, and combinations of the foregoing. For example, the system 40 in the context of probe 30 being an OCT probe can include the sample arm of an interferometer, the reference arm of an interferometer, photodiodes, a control system, and patient interface unit. Similarly, as another example, in the context of an IVUS system, the intravascular data collection system 40 can include ultrasound signal generating and processing circuitry, noise filters, rotatable joint, motors, and interface units. In one embodiment, the data collection system 40 and the angiography system 20 have a shared clock or other timing signals configured to synchronize angiography video frame time stamps and OCT image frame time stamps. In addition to the invasive and noninvasive image data collection systems and devices of FIG. 1, various other types of data can be collected with regard to region 25 of the subject and other parameters of interest of the subject. For example, the data collection probe 30 can include one or more pressure sensors such as for example a pressure wire. A pressure wire can be used without the additions of OCT or ultrasound components. Pressure readings can be obtained along the segments of a blood vessel in region 25 of the subject 4.

One or more displays 82, 83 can also be used to show the various workflows disclosed herein, VFR calculations, calcium angles, EEL detections, calcium detections, proximal frames, distal frames, and associated graphical user interfaces, EEL-based metrics, stent/no stent decisions, scores, recommendations for debulking and other procedures, evidence based recommendations informed by automatic detection of regions/features of interest, an angiography frame of data, an OCT frame, image data, stent planning interfaces, morphology interfaces, review interfaces, stent deployment interfaces, user interfaces for OCT and angiography data and other controls and features of interest. Two exemplary workflows, workflow A, and workflow B may be displayed on displays 82, 83 and may include any of the graphical user interfaces, panels, arterial images, arterial representations, features of interest, regions of interest, and other measurements and graphical elements disclosed or depicted herein, include any subsets thereof, without limitation.

The intravascular image data such as the frames of intravascular data generated using the data collection probe 30 can be routed to the data collection processing system 40 coupled to the probe via PIU 35. The noninvasive image data generated using image management system 22 can be transmitted to, stored in, and processed by one or more servers or workstations such as the co-registration server 50 workstation 85. A video frame grabber device 55 such as a computer board configured to capture the angiography image data from system 22 can be used in various embodiments.

In one embodiment, the server 50 includes one or more co-registration software modules 67 that are stored in memory 70 and are executed by processor 80. The server may include a trained neural network 52 suitable for implementing various embodiments of the disclosures. In one embodiment, an AI processor, such as a graphical processing unit, 53 is included in the server 50 and in electrical communication with memory 70. The computing device/server 50 can include other typical components for a processor-based computing server. Alternatively, more databases such as database 90 can be configured to receive image data generated, parameters of the subject, and other information generated, received by or transferred to the database 90 by one or more of the systems devices or components shown in FIG. 1.

Although database 90 is shown connected to server 50 while being stored in memory at workstation 85, this is but one exemplary configuration. For example, the software modules 67 can be running on a processor at workstation 85 and the database 90 can be located in the memory of server 50. The device or system use to run various software modules are provided as examples. In various combinations the hardware and software described herein can be used to obtain frames of image data, process such image data, and register such image data.

As otherwise noted herein, the software modules 67 can include software such as preprocessing software, transforms, matrices, and other software-based components that are used to process image data or respond to patient triggers to facilitate co-registration of different types of image data by other software-based components 67 or to otherwise perform annotation of image data to generate ground truths and other software, modules, and functions suitable for implementing various embodiments of the disclosure. The modules can include workflows, morphology workflow, review workflow, sizing workflow, deployment workflow, computer-directed workflow, computer-support workflow, lumen detection using a scan line based or image based approach, workflows, indicia generation, VFR computations, calcium angle/arc generation, stent detection using a scan line based or image based approach, indicator generation, apposition bar generation for stent planning, proximal/distal color coding/indicia generation, lumen boundary detection, stent expansion, lumen profile, target lumen profile, side branches and missing data, and others.

The database 90 can be configured to receive and store angiography image data 92 such as image data generated by angiography system 20 and obtained by the frame grabber 55 server 50. The database 90 can be configured to receive and store intravascular image data such as OCT image data, IVUS image data, infrared spectroscopy image data, or OFDI image data, or other non-intravascular arterial image data 95 such as image data generated by OCT system 40 and obtained by the frame grabber 55 of server 50.

In addition, the subject 4 can be electrically coupled via one or more electrodes to one more monitors such as, for example, monitor 49. Monitor 49 can include without limitation an electrocardiogram monitor configured to generate data relating to cardiac function and showing various states of the subject such as systole and diastole.

The use of arrow heads showing directionality in a given figure or the lack thereof are not intended to limit or require a direction in which information can flow. For a given connector, such as the arrows and lines shown connecting the elements shown in FIG. 1, for example, information can flow in one or more directions or in only one direction as suitable for a given embodiment. The connections can include various suitable data transmitting connections such as optical, wire, power, wireless, or electrical connections.

One or more software modules can be used to process frames of angiography data received from an angiography system such as system 22 shown in FIG. 1. Various software modules that can include without limitation software, a component thereof, or one or more steps of a software-based or processor executed method can be used in a given embodiment of the disclosure.

In part, the disclosure relates to intravascular data collections systems and related methods by which intravascular data collected by an intravascular probe can be transformed or analyzed by a processor-based system. The results of such analysis and transformation can be displayed to an end user in various representations such as a display that is in communication with a pipeline of imaging processing software modules for image segmentation/detection of features or regions of interest relative to image data, a machine learning system having a neural network to classify components of a medical image and detect instances of features and regions of interest, and other image processing and segmentation/detection systems. In one embodiment, a given imaging system, such as an OCT, IVUS, infrared spectroscopy, x-ray based imaging system is in electronic communication with an MLS and able to display modified versions of the image data obtained using a given type of imaging system during the same session when such image data was obtained. Various neural network architectures may be used for image segmentation such as V-net, U-net, CUMedVision1, CUMedVision2, VGGNet, Multi-stage Multi-recursive-input Fully Convolutional Networks (M²FCN) Coarse-to-Fine Stacked Fully Convolutional Net, Deep Active Learning Framework, ResNet, combinations thereof, and other neural networks and software-based machine learning frameworks suitable for image segmentation.

System 5 of Fig. 1 may be configured to perform VFR computations using anatomical information that can be obtained through imaging of the blood vessel both through intravascular images and extravascular images. For example, the intravascular OCT images collected during a vessel pullback procedure may be co-registered with extravascular angiographic images of the same. During an OCT pullback procedure, an intravascular imaging probe is pulled through a region of the blood vessel, and often, the intravascular imaging will only occur over a portion of the entire blood vessel. Without additional information regarding the structure of the blood vessel at the regions that have not been imaged by an intravascular probe, assumptions are needed in order to perform VFR computations. For example, the VFR computation may be based on an assumption that the intravascular images were captured over a region that corresponds to roughly the center of the vessel, so as to have equal proximal and distal epicardial length, and so as to have proximal and distal epicardial vessel sizes that are similar to the proximal and distal vessel sizes of the OCT imaged segment.

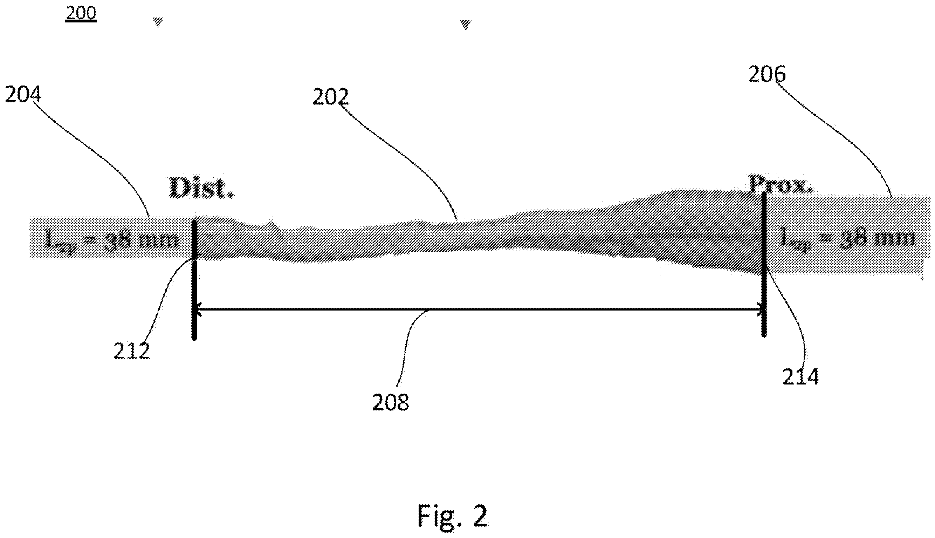

In Fig. 2, a blood vessel 200 is shown to have three different segments 202, 204, and 208 along its longitudinal length (segments not to scale). Segment 202 is the portion of blood vessel 200 that has been imaged by an intravascular OCT probe. Distal location 212 is the location within blood vessel 200 at which the OCT probe began to capture images as part of the pullback procedure, and proximal location 214 is the location within blood vessel 200 at which the OCT probe stopped capturing images at the end of the pullback procedure. Segment 204 is the distal epicardial segment that extends distally from location 212 of segment 202, and segment 206 is the proximal epicardial segment that extends proximally from location 214 of segment 202. Segment 202 is shown to vary in size along the longitudinal axis of blood vessel 200. This represents the manner in which the cross-sectional diameter and area of the inner lumen of blood vessel 200 changes along the longitudinal axis of segment 202. The cross-sectional diameter and area along segment 202 is identified based on analysis of a plurality of OCT images that are captured along the length of segment 202. However, such OCT images are not available for segments 204 and 206 of blood vessel 200. Instead, segments 204 and 206 are represented based on approximations regarding the typical length and cross-sectional size of the lumen along segments 204 and 206.

As shown in Fig. 2, the lengths of distal epicardial segment 204 and proximal epicardial segment 206 have been approximated to each be 38 mm. This approximation could be based, at least in part, on the average vessel lengths, as provided in medical literature, or as determined from a population of patients. For example, if blood vessel 200 is a left anterior descending ("LAD") artery, the disclosed systems and methods may determine that the typical LAD artery has a length that is approximately 76 mm longer than the length of the OCT pullback. As stated above, an assumption may be made that the region of the OCT pullback occurred roughly in the center of the vessel, so that the distal and proximal segments 204 and 206 are each shown as being 38 mm. Similarly, the width, or cross-sectional diameter, of distal and proximal segments 204 and 206 are shown as corresponding to the width of the blood vessel at locations 212 and 214, respectively. Accordingly, an assumption is made that the vessel's cross-sectional diameter remains constant as it extends from the intravascularly imaged segment 202. However, to the extent that vessel 200 significantly differs from these assumptions, the use these assumptions could produce an inaccurate VFR calculation.

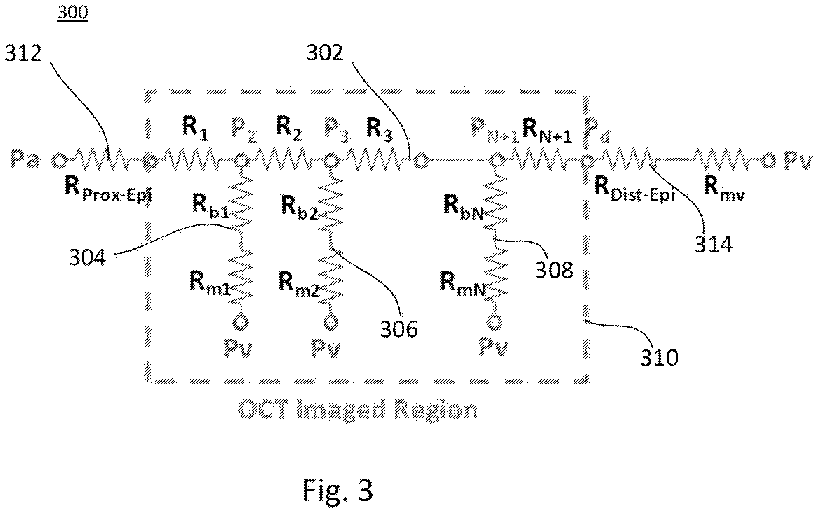

In Fig. 3, a flow resistance model 300 of a blood vessel 302 is shown, which can be used to compute the VFR of blood vessel 302. Box 310 delineates the region of blood vessel 302 that has been imaged by an intravascular OCT probe. As can be seen in model 300, the OCT imaged region of vessel 302 includes vessel branches 304, 306, and 308. The flow resistance of vessel 302 can be computed using the OCT images of vessel 302, which can be used to identify the length of the vessel 302 as well as the cross-section diameter or area of vessel 302. The location and cross-sectional diameter or area of the side branches 304, 306, and 308 can also be identified using the OCT images. The resisters, such as R₁, R₂,...R_{N+1}, represent the relative pressure drop that occurs along segments of the blood vessel 302 based on the segment's length and cross-sectional size. Similarly, the resisters located along side branches 304, 306, and 308 represent relative pressure drops that occur due to the location and size of the respective side branches. Blood vessel 302 also includes a proximal segment and distal segment that were not imaged by an OCT probe. These segments are outside of box 310. However, as represented by resisters 312 and 314, these segments have an effect on the computed pressure drops that occur between the aortic pressure ("Pa") and the distal-end pressure ("Pv"). disclosure, an estimation of the proximal resistance (resister 312) and distal resistance (resister 314) can be determined using information obtained from extravascular images, thereby improving the VFR computation relative to a VFR computation that uses generic assumptions regarding the size of vessel 302 outside of the intravascularly imaged region.

In addition, the resistance values used for a VFR computation may include a representation of microvascular resistance. For example, as shown in Fig. 3, a resistance Rₘᵥ may be used to represent the microvascular resistance of a peripheral coronary vascular bed. An estimation of the microvascular resistance can be determined using information obtained from one or more extravascular images. As set forth below, information relating to the length of the OCT pullback may be used in combination with the extravascular images, so as generate a more accurate determination of the proximal resistance, distal resistance, and microvascular resistance within the vessel. With regard to the microvascular resistance, it is possible to determine resistance based on the maximum blood flow that can be achieved in the vessel when the pressure drop across the epicardial arteries is negligible. For example, a hyperemic velocity value may be used for determining the microvascular resistance. In addition, a hyperemic microvascular resistance index can be determined and used in connection with a VFR computation. The determination of microvascular resistance, including a hyperemic velocity and hyperemic microvascular resistance index, is provided in U.S. Application 13/796,710.

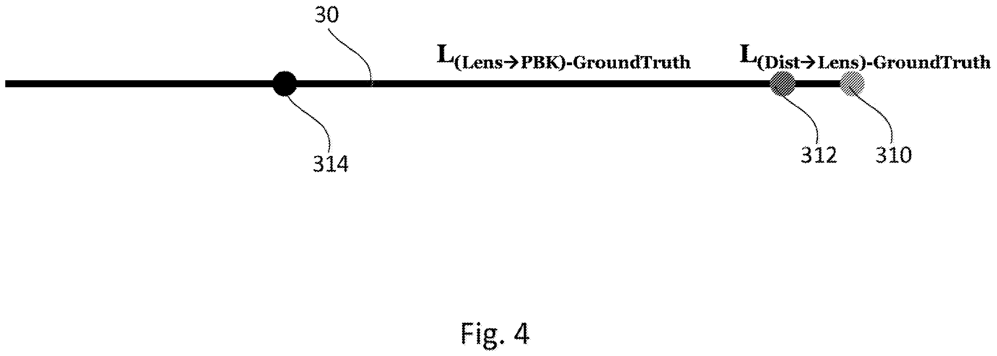

System 5 of Fig. 1 may be configured so that catheter-based data collection probe 30 includes one or more radiopaque markers. For example, in Fig. 4, catheter-based data collection probe 30 contains three radiopaque markers along its length. Marker 310 is a distal marker that is located on the distal end of probe 30. Marker 312 is a lens marker that is located at the lens that is used to capture the intravascular images. Marker 314 is a pullback marker which can be proximally located at a position on the catheter that corresponds to the position at or near where the distal end of probe 30 will be at the end of the pullback procedure. The distance between lens marker 312 and pullback marker 314 is designated in Fig. 4 as L_{(Lens→PBK)-Groundtruth} while the distance between distal marker 310 and lens marker 312 is designated as L_{(Dist→Lens)-Groundtruth}. These lengths are referred to as a "groundtruth" as they correspond to the actual distances between the markers.

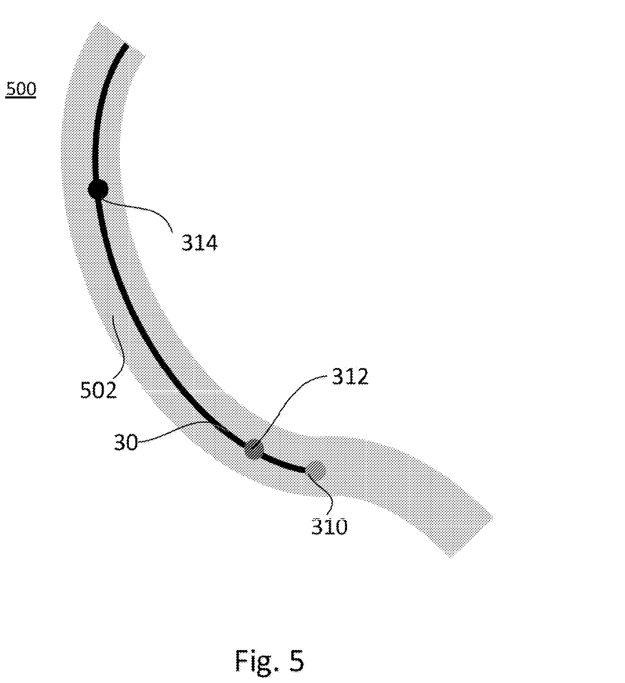

Fig. 5 is a representation of an angiographic image 500 in which probe 30 has been placed within vessel 502. When probe 30 is placed being positioned within vessel 502 the distal end of probe 30 can be positioned at an initial pullback location within vessel 502. The initial pullback location corresponds to the location of probe 30 when the pullback procedure is to be initiated. Prior to contrast fluid being introduced into vessel 502, angiographic image 500 of vessel 502 may be acquired when probe 30 is in the initial pullback location shown in Fig. 5. System 5 of Fig. 1, is configured to identify the location of markers 310, 312, and 314 within angiographic image 500 when probe 30 is in the initial pullback location. Once the pullback is initiated, system 5 is also configured to track the position of the probe markers 310, 312, and 314 within subsequent angiographic images. System 5 can then calculate the apparent distance that one or more of the markers travels within vessel, as represented in the angiographic images.

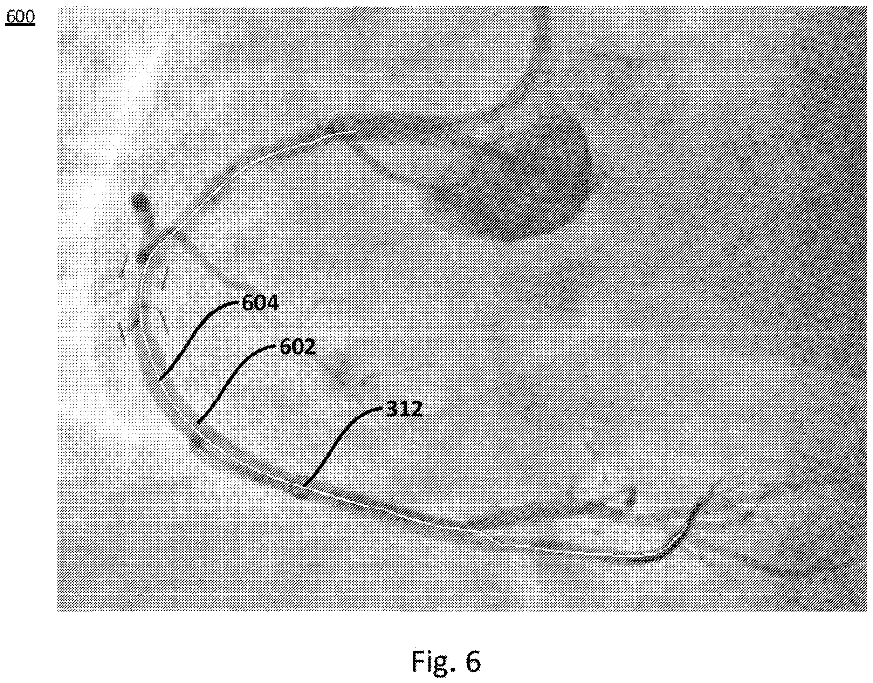

Fig. 6 is an angiographic image 600 that includes a centerline 604 running through vessel 602. The position of marker 312 within vessel 602 is shown. This position corresponds to the intial pullback position of marker 312 prior to initiating the pullback proceedure. Various methods for computing accurate vessel centerlines may be used, such as a fast marching algorithm and a narrow band method. Whichever approach is used, the disclosed system may identifiy and display centerlines for both the main vessel and any identified side branches.

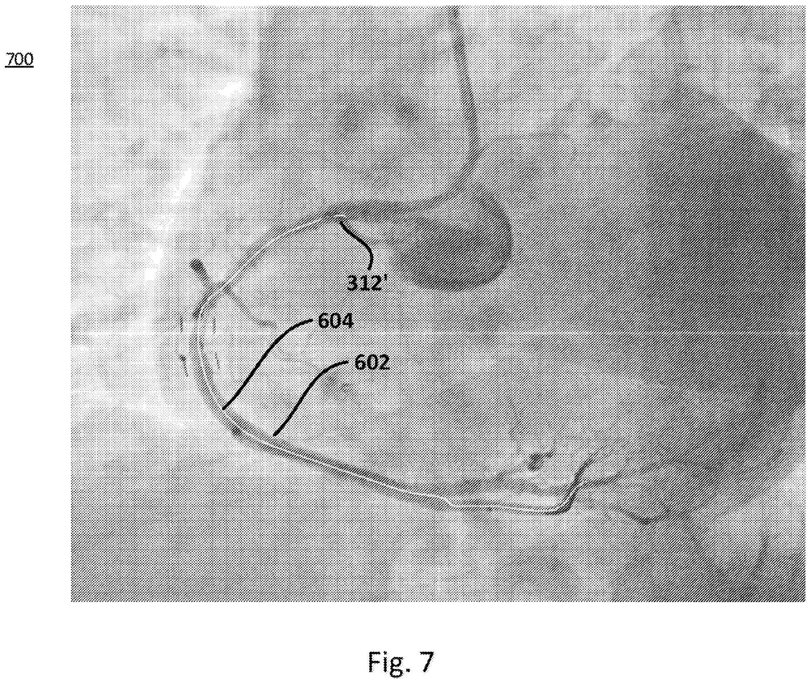

Fig. 7 is an angiographic image 700 of vessel 602 once the pullback proceedure has occurred, thus the position of marker 312' within vessel 602 has changed, as the probe has been pulled through the vessel. To determine the apparent pullback length within the angiographic images, the disclosed system may deformably register one or more of the subsequent angiographic images to the initial angiographic image that was captured when the probe was in the initial pullback position.

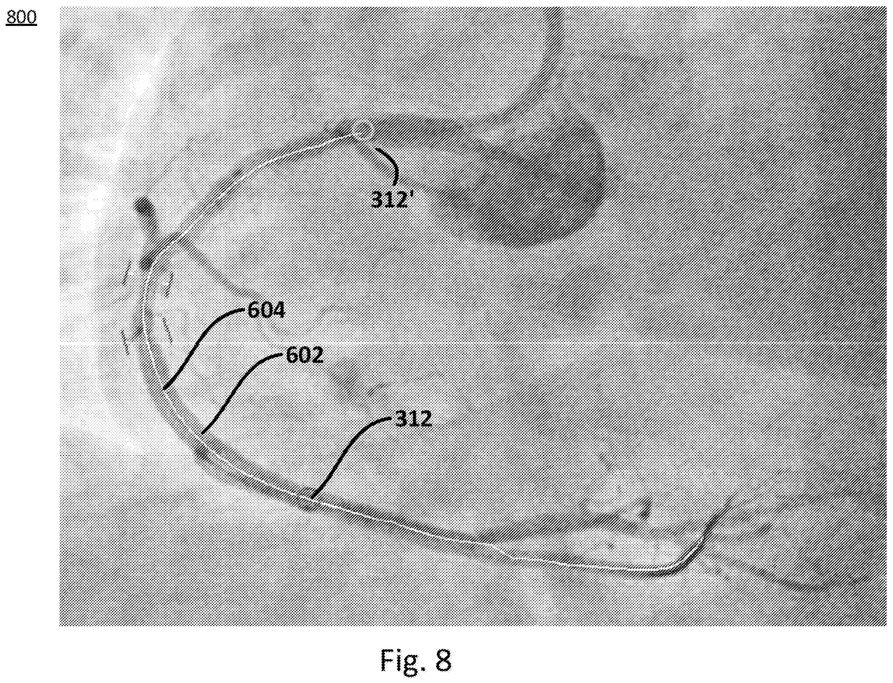

For example, Fig. 8 is a modified angiographic image 800 that has modified the original angiographic image 600 to include the position of marker 312' from angiographic image 700. The distance between marker 312 and marker 312', as meassured along centerline 604, can be referred to as L_{pullback-angio}, as it represents the apparent length of the pullback within the angiographic images. The length L_{pullback-angio} can be compared with the actual pullback length, L_{pullback-groundtruth}, so as to determine the degree to which the depth and orientation of the vessel is effecting the projected length of the vessel within the angiographic images. The depth and orientation of the vessel can cause foreshortening of the apparent vessel length, so that the length L_{pullback-angio} will often be less than L_{pullback-groundtruth}. By correlating L_{pullback-groundtruth} with distance between marker 312 and marker 312' in angiographic image 800, the disclosed system can determine account for the depth and orientation of the vessel so as to identify a true vessel length within the angiographic image. The same analysis can also be performed between different markers of the same image. For example, the length L_{(Lens→PBK)-Groundtruth} described above can be correlated with the distance, along the vessel centerline, between the pullback marker 310 and the lens marker 312, as viewed within the angiographic image. Similarly, the length L_{(Dist→Lens)-Groundtruth} can be correlated with the distance, along the vessel centerline, between the lens marker 312 and the distal marker 314.

Returning to system 5 shown in Fig. 1, the angiographic system 20 may be configured to take a plurality of angiographic images from a plurality of different positions relative to subject 4. In capturing the plurality of angiographic images, system 5 may determine the three-dimensional orientation of regions of interest, such as by identifying the position and takeoff angle of side branches from of a particular vessel. The side branch positions may be identified based on the centerline computations discussed above. U.S. Patent 10,172,582 also discloses systems and methods for identifying the position and orientation of vessel bifurcations within image frames, and is incorporated herein by reference for these purposes.

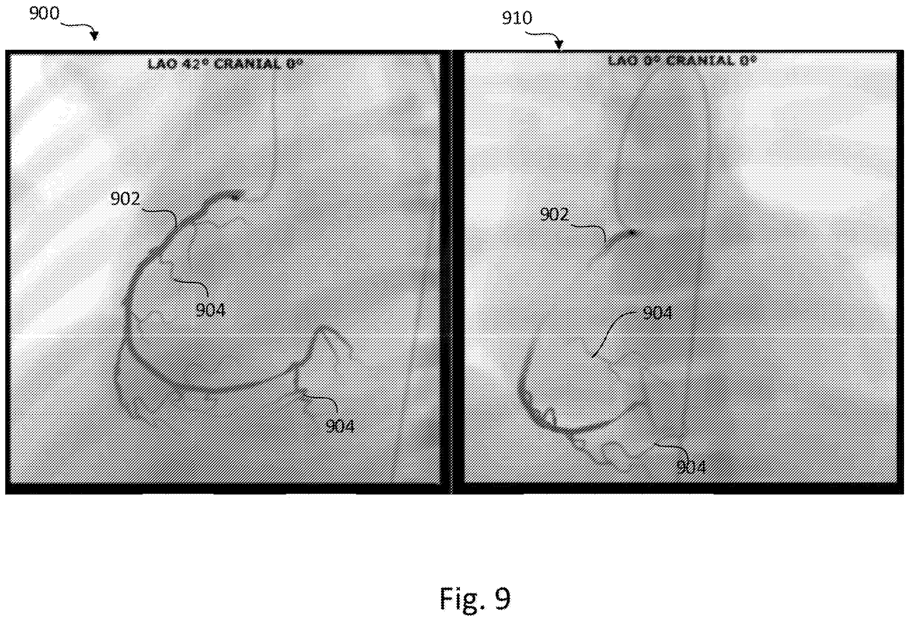

In Fig. 9, two different angiographic images 900 and 910 are displayed, with each containing an image of vessel 902 from two different angles. As indicated in angiographic image 900, the image has been captured at a left anterior oblique anglulation of 42 degrees and a cranial angulation of zero degrees. In image 910, the angiographic image has been captured at a left anterior oblique angulation of zero degrees and a cranial angulation of zero degrees. A plurality of angiographic images, like images 900 and 910, may be used to determine the orientation of vessel 902, as well as branch vessels 904 that branch off of vessel 902. For example, the x-ray source and detector of the angiographic system may be moved around the patient to capture angiographic images at various lateral, cranial, and caudal anglulations. These multiangle angiographic images may then be processed by the system to generate a three-dimensional model of the vessels and surrounding structures.

In generating the three-dimensional model or in coregestering the angiographic images to the intravascular images, a determination may be made as to which angiographic image frames should be used. For example, the angiographic images may be captured over a plurality of cario cycles, and angiographic images may be sellected so as to analyze image frames that correspond to the same or similar portions of the patient's cardio cycle.

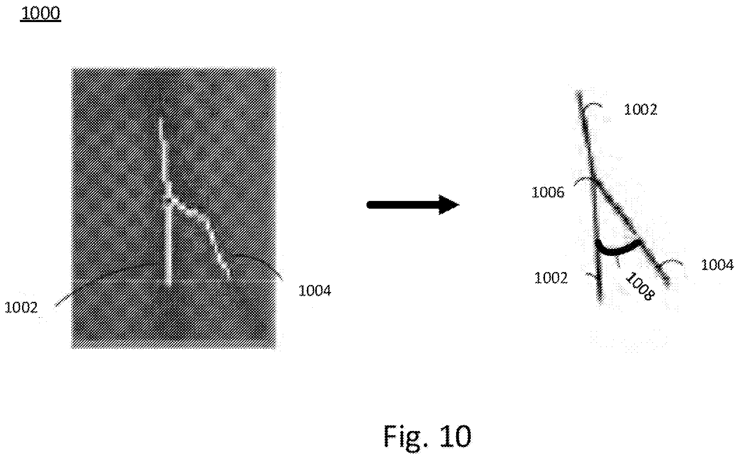

In Fig. 10, a portion of angiographic image 1000 is shown in which the centerline of vessel 1002 has been identified, as well as the centerline of a branch vessel 1004. As stated above, a plurality of angiographic images may be taken at different lateral, cranial, and caudal angulations, may be similarly analyzed to identify the centerlined 1002 and 1004 in those images as well. A three-dimensional model of the vessel may then be generated using the multiangled angiographic images, so as to identify the orientation of the centerline of branch vessel 1004 relative to the centerline of vessel 1002. The orientation of the branch vessel may be identified using an identified location and takeoff angle of the branch vessel. For example, in Fig. 10 the centerline of vessel 1002 and the centerline of vessel 1004 are used to generate a three-dimensional model that can be used to identify a position 1006 at which the branch occurs, as well as the takeoff angle 1008 of the branch from the vessel.

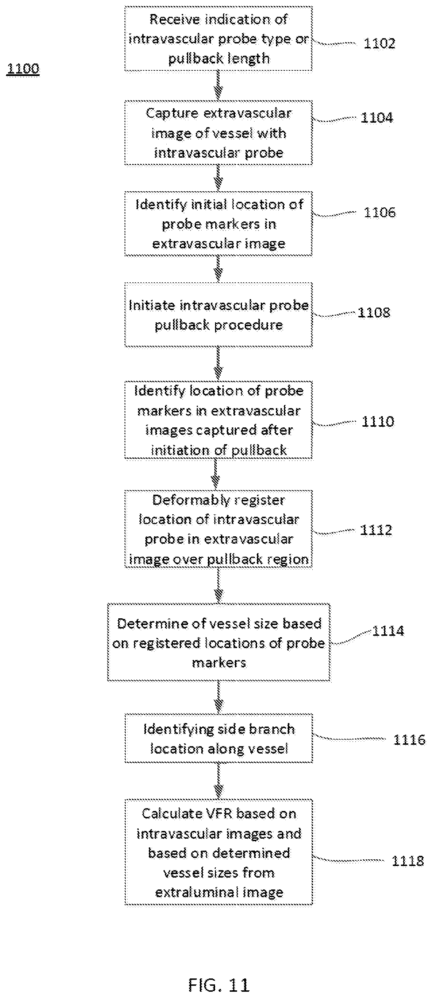

As discussed above, one or more processors of system 5 can be configured to analyze intravascular and extravascular images to determine the VFR of particular regions of the imaged blood vessel. Image processing techniques and/or machine learning may compute VFR. The frames of the pullback may be stretched and aligned using various windows or bins of alignment features. This image data can be presented using various graphical user interfaces. FIG. 11 is a flowchart 1100 that includes functions in accordance with aspects of the disclosure. For example, functions of flowchart 1100 may be performed using one or more processors of the disclosed system to improve the calculation of the VFR of a blood vessel.

In Block 1102, a system may receive an indication of an intravascular probe type or a pullback length that will be used in capturing intravascular images of a blood vessel. The indication of the intravascular probe or the pullback length may be provided by a user of the system. For example, the user may select from a plurality of intravascular probe types. The system may identify the pullback length by accessing stored data regarding the identified probe type or the user may provide input that identifies a particular pullback length that will be used in capturing the intravascular images. In Block 1104, one or more extravascular images are captured of the vessel and the intravascular probe within the vessel. This extravascular image may be an angiographic image of the vessel. One or more of the extravascular images can be analyzed so as to identify the initial location of one or more probe markers within the extravascular image (Block 1106). As discuss above, the intravascular probe may contain one or more radiopaque markers, and the position of these markers may be identified when the intravascular probe has been placed into an initial position for the pullback procedure.

In Block 1108, the pullback procedure is initiated. This may include flushing at least a portion of the vessel with contrast fluid and capturing intravascular images over the length of the pullback of the intravascular probe. The location of the one or more markers of the intravascular probe may then be identified after the probe has traversed a portion of the vessel as part of the pullback procedure. (Block 1110). In Block 1112, the positions of the intravascular probe is deformably co-registered with the extravascular images. As discussed above, the "ground truth" pullback length of the intravascular probe can be associated with the apparent distance that the probe has travelled within the intravascular images. In particular, the initial and final location of the one or more probe markers can be mapped onto a single extravascular image as part of the deformable co-registration. Based on this mapping, a determination may be made of the vessel size. (Block 1114).

This determination of the vessel size can include regions beyond those that were imaged by the intravascular probe. For example, the entire length of the vessel may be extrapolated based on the known distance that the probe markers travelled during the pullback procedure. In addition, the cross-sectional diameters and areas of the vessel can be extrapolated by comparing the apparent width of the vessel in the deformably co-registered extravascular images with the known diameters and areas for the region of the vessel that has been imaged by the intravascular probe. The vessel length within the extravascular image may be estimated by identifying a lumen centerline and by dividing the vessel into three segments: proximal epicardial, intravascularly imaged, and distal epicardial segments. The intravascularly imaged segment will correspond with the "ground truth" distance that the intravascular probe travelled. The length of the proximal epicardial and distal epicardial segments may be determined by comparing their apparent length in one or more extravascular images with the apparent length of the intravascularly imaged segment. The ratio of the length of the compared segments can be used with the "ground truth" distance of the intravascularly imaged segment to determine the actual length of the proximal epicardial and distal epicardial segments. Accordingly, the intravascularly imaged segment can be used for scaling the proximal epicardial and distal epicardial segments.

The cross-sectional size of the vessel can also be determined by analyzing the contrast boundary of the vessel lumen to determine a cross-sectional width of the vessel within an extravascular image. This cross-sectional width may be correlated with the known diameter of the vessel based on the intravascular images, and the cross-sectional diameter of the vessel may then be extrapolated for the proximal epicardial and distal epicardial segments as well. For example, the intravascular images captured at the distal and proximal ends of the pullback can be used to determine the lumen diameter at these locations. The determined diameter at these locations can then be used for scaling the cross-sectional diameters of the vessel within extravascular image, including the cross-sectional diameters along the proximal epicardial and distal epicardial segments. The vessel's size, including length and cross-sectional size, may therefore be determined for all three vessel segments.

In Block 1116, the extravascular images and intravascular images may be analyzed to determine the location and size of vessel side branches based on identification of bifurcations along the centerline of the lumen for the imaged vessel. These bifurcations at the proximal and distal epicardial segments may be used to identify location markers for vessel tapering. Additionally, the size of microvasculature that extends from the vessel can also be determined by comparing the length and width data of the intravascular images with the microvasculature that is visible within the extravascular image. As discussed above, this microvasculature can be treated as another resistance value that is a part of the analyzed vessel segments.

The determined size of the vessel, as well as the size and location of the side branches, may then be used to computer the VFR of the vessel (Block 1118). For example, the sizes of the three vessel segments, as determined by the extravascular images, may be introduced, along with information collected from the intravascular images, into a flow resistance model, such as model 300 of Fig. 3 that is used to calculate the pressure drops across regions of the vessel.

The blocks of flowchart 1100 may be performed using angiographic images that are captured from a single location. However multiangled angiographic images may also be used in connection with flowchart 1100. For example, Block 1104 may include capturing a plurality of angiographic images at a plurality of angulations relative to the patient. In addition, block 1114 may include determining the vessel size based at least in part on a three-dimensional model of the vessel that is based on the multiangled angiographic images. For example, the lumen diameter of the vessel may be estimated by detecting the distance of the contrast boundary in the angiographic image from the identified centerline of the vessel. In addition, the location of the side branches that are identified in Block 1116 may include an analysis of the three-dimensional model to determine the takeoff angle of each side branch. Thus, the orientation of the identified side branches, including the location and takeoff angle of each side branch, may be used as part of the VFR calculation that occurs at block 1118.

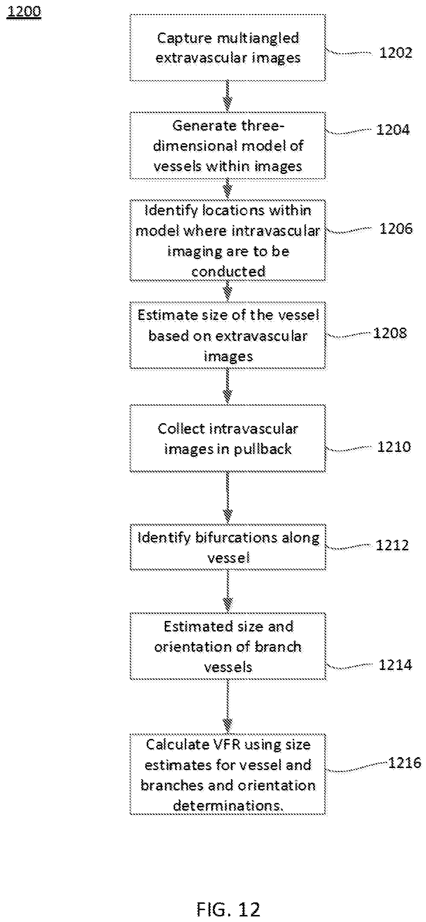

Fig. 12 is a flowchart 1200 in which the disclosed systems collect and analyze multiangled extravascular images. In Block 1202, multiangled extravascular images are captured, and in Block 1204, a three-dimensional model of one or more imaged vessels is generated based on the multiangled extravascular images. A user may then identify a proximal and distal location within an imaged vessel that represents the proximal and distal locations between which an intravascular pullback procedure is to be conducted (Block 1206). For example, the user may identify the proximal and distal location on one of the displayed extravascular images or on a display of the generated three-dimensional model. The length between the proximal and distal locations can be displayed to the user based on analysis of the length within the three-dimensional model.

In Block 1208, the size of the vessel may be estimated, including the size of the vessel in the regions distal and proximal to the region to be intravascularly image. This estimate may be based on identification of centerlines of the vessel, as determined by tracing the contrast boundaries within the lumen of the vessel, and may include both an estimate of the vessel length and the vessel diameter/area. For example, the vessel diameter may be estimated by measuring the radial distance of the contrast boundary within the extravascular images of the vessel, as analyzed within the generated three-dimensional model. Intravascular images are collected in Block 1210 as part of a pullback procedure between the identified proximal and distal locations. The intravascular images are coregistered with the extravascular images, and may be incorporated into the three-dimensional model data. In Block 1212, bifurcations along the centerline of the imaged vessel are identified. The size and orientation of the branch vessels corresponding to the identified bifurcations may then be estimated (Block 1214). As discussed above, the orientation of the branch vessel may include identification of the location of the bifurcation, as well as the takeoff angle of the branch vessel, as determined by the identified centerline of the bifurcation. Additionally, the size of microvasculature that extends from the vessel can also be determined by comparing the length and width data of the intravascular images with the microvasculature that is visible within the extravascular image. As discussed above, this microvasculature can be treated as another resistance value that is a part of the analyzed vessel segments. The VFR of the vessel is calculated in Block 1216. This VFR calculation may use any set or subset of the estimated vessel and branch sizes and orientations, as determined by the analysis of the extravascular images. For example, the proximal and distal lengths of the vessel outside of the intravascularly-imaged region, including the vessel's microvasculature, may be incorporated into a resistance model that is used to calculate the VFR of the vessel. The size, location, and takeoff angle of each branch vessel may also be incorporated into the resistance model used for the VFR calculation.

Some portions of the detailed description are presented in terms of methods such as algorithms and symbolic representations of operations on data bits within a computer memory. These algorithmic descriptions and representations can be used by those skilled in the computer and software related fields. In one embodiment, an algorithm is here, and generally, conceived to be a self-consistent sequence of operations leading to a desired result. The operations performed as methods stops or otherwise described herein are those requiring physical manipulations of physical quantities. Usually, though not necessarily, these quantities take the form of electrical or magnetic signals capable of being stored, transferred, combined, transformed, compared, and otherwise manipulated.

The algorithms and displays presented herein are not inherently related to any particular computer or other apparatus. Various general purpose systems may be used with programs in accordance with the teachings herein, or it may prove convenient to construct more specialized apparatus to perform the required method steps. The required structure for a variety of these systems will appear from the description below.

The aspects, embodiments, features, and examples of the disclosure are to be considered illustrative in all respects and are not intended to limit the disclosure, the scope of which is defined only by the claims. Other embodiments, modifications, and usages will be apparent to those skilled in the art without departing from the scope of the claimed invention.

The use of headings and sections in the application is not meant to limit the invention; each section can apply to any aspect, embodiment, or feature of the invention.

Throughout the application, where compositions are described as having, including, or comprising specific components, or where processes are described as having, including or comprising specific process steps, it is contemplated that compositions of the present teachings also consist essentially of, or consist of, the recited components, and that the processes of the present teachings also consist essentially of, or consist of, the recited process steps.

In the application, where an element or component is said to be included in and/or selected from a list of recited elements or components, it should be understood that the element or component can be any one of the recited elements or components and can be selected from a group consisting of two or more of the recited elements or components. Further, it should be understood that elements and/or features of a composition, an apparatus, or a method described herein can be combined in a variety of ways without departing from the scope of the present teachings, whether explicit or implicit herein.

The use of the terms "include," "includes," "including," "have," "has," or "having" should be generally understood as open-ended and non-limiting unless specifically stated otherwise.

The use of the singular herein includes the plural (and vice versa) unless specifically stated otherwise. Moreover, the singular forms "a," "an," and "the" include plural forms unless the context clearly dictates otherwise. In addition, where the use of the term "about" is before a quantitative value, the present teachings also include the specific quantitative value itself, unless specifically stated otherwise. As used herein, the term "about" refers to a ±10% variation from the nominal value. All numerical values and ranges disclosed herein are deemed to include "about" before each value.

It should be understood that the order of steps or order for performing certain actions is immaterial so long as the present teachings remain operable. Moreover, two or more steps or actions may be conducted simultaneously, and steps may be removed or replaced in accordance with aspects of the disclosure.

Where a range or list of values is provided, each intervening value between the upper and lower limits of that range or list of values is individually contemplated and is encompassed within the invention as if each value were specifically enumerated herein. In addition, smaller ranges between and including the upper and lower limits of a given range are contemplated and encompassed within the invention. The listing of exemplary values or ranges is not a disclaimer of other values or ranges between and including the upper and lower limits of a given range.

## Claims

1. A computer implemented method for identifying attributes of a blood vessel (200, 302) using a plurality of extravascular images of a vessel taken during a pullback of an intravascular imaging probe having a defined pullback length along a first region of the vessel which was imaged by the intravascular imaging probe during the pullback, the method comprising:
detecting, by the one or more processors, locations of one or more markers (312, 312') in the plurality of extravascular images;
comparing, by the one or more processors, an apparent length of the first region (208) of the vessel (202) with the defined pullback length, the apparent length being represented in the plurality of extravascular images by locations of the one or more markers (312, 312') in the plurality of extravascular images; and
determining, by the one or more processors, a size of a second region (204, 206) of the vessel represented in the plurality of extravascular images but not imaged by the intravascular probe during the pullback, using a result of the comparison of the apparent length of the first region of the vessel with the defined pullback length.

2. The method of claim 1, wherein the size of the second region (204, 206) of the vessel includes at least one of a length of the second region, a cross-section diameter within the second region, and a cross-sectional area within the second region.

3. The method of claim 1, further comprising computing, by the one or more processors, a virtual flow reserve, VFR, of the vessel based on a plurality of images captured by the intravascular imaging probe and based on the determined size of the second region of the vessel.

4. The method of claim 3, wherein the VFR of the vessel is computed based on a distance between a vessel centerline (604) and a boundary of the vessel the second region of the vessel identified in at least one of the plurality of extravascular images.

5. The method of claim 1, wherein the second region of the vessel 202 includes at least one of a distal epicardial region (204) and a proximal epicardial region (206) of the vessel.

6. The method of claim 5, wherein determining a size of a second region of the vessel includes determining a length of the distal epicardial region (204) and a length of the proximal epicardial region (206).

7. The method of claim 1, wherein correlating the first region of the vessel represented in the plurality of extravascular images with the defined pullback length includes scaling a lumen size represented in at least one of the plurality of the extravascular images.

8. The method of claim 1, wherein the plurality of extravascular images are taken from a plurality of locations relative to the vessel.

9. The method of claim 8, further comprising analyzing, by the one or more processors, the plurality of extravascular images so as to identify a three-dimensional orientation of one or more objects relative to the vessel.

10. The method of claim 9, wherein the one or more objects comprises a branch from the vessel and wherein the three-dimensional orientation of the branch includes a position and takeoff angle of the branch relative to the vessel.

11. The method of claim 10, further comprising computing, by the one or more processors, a virtual flow reserve, VFR, of the vessel based on the identified position and takeoff angle of the branch.

12. A system for identifying attributes of a blood vessel, the system being arranged to carry out the method steps of any preceding claim.

13. A computer program comprising instructions which, when the program is executed by a computer, cause the computer to carry out the steps of any of claims 1 to 11.

## Patentansprüche

1. Computerimplementiertes Verfahren zum Bestimmen von Attributen eines Blutgefäßes (200, 302) unter Verwendung einer Vielzahl von extravaskulären Bildern eines Gefäßes, die während eines Rückzugs einer intravaskulären Bildgebungssonde mit einer festgelegten Rückzugslänge entlang einer ersten Region des Gefäßes, die von der intravaskulären Bildgebungssonde während des Rückzugs abgebildet wurde, aufgenommen wurden, wobei das Verfahren umfasst:
Ermitteln, durch den einen oder mehrere Prozessoren, von Positionen eines oder mehrerer Marker (312, 312') in der Vielzahl von extravaskulären Bildern;
Vergleichen, durch den einen oder mehrere Prozessoren, einer sichtbaren Länge der ersten Region (208) des Gefäßes (202) mit der festgelegten Rückzugslänge, wobei die sichtbare Länge in der Vielzahl von extravaskulären Bildern durch Positionen des einen oder mehrerer Marker (312, 312') in der Vielzahl von extravaskulären Bildern dargestellt wird; und
Bestimmen, durch den einen oder mehrere Prozessoren, einer Größe einer zweiten Region (204, 206) des Gefäßes, die in der Vielzahl von extravaskulären Bildern dargestellt ist, jedoch nicht durch die intravaskuläre Sonde während des Rückzugs abgebildet wird, indem ein Ergebnis des Vergleichs der sichtbaren Länge der ersten Region des Gefäßes mit der festgelegten Rückzugslänge verwendet wird.

2. Verfahren nach Anspruch 1, wobei die Größe des zweiten Bereichs (204, 206) des Gefäßes zumindest entweder eine Länge der zweiten Region, einen Querschnittsdurchmesser in der zweiten Region, oder eine Querschnittsfläche in der zweiten Region umfasst.

3. Verfahren nach Anspruch 1, das weiter das Berechnen, durch den einen oder mehrere Prozessoren, einer virtuellen Flussreserve, VFR, des Gefäßes auf der Basis einer Vielzahl von Bildern, die von der intravaskulären Bildgebungssonde aufgenommen werden, und auf der Basis der bestimmten Größe der zweiten Region des Gefäßes umfasst.

4. Verfahren nach Anspruch 3, wobei die VFR des Gefäßes auf der Basis eines Abstands zwischen einer Gefäß-Mittellinie (604) und einem Rand des Gefäßes der zweiten Region des Gefäßes, der in mindestens einem der Vielzahl von extravaskulären Bildern bestimmt wird, berechnet wird.

5. Verfahren nach Anspruch 1, wobei die zweite Region des Gefäßes (202) mindestens entweder eine distale epikardiale Region (204) oder eine proximale epikardiale Region (206) des Gefäßes umfasst.

6. Verfahren nach Anspruch 5, wobei das Bestimmen einer Größe der zweiten Region des Gefäßes das Bestimmen einer Länge der distalen epikardialen Region (204) und einer Länge der proximalen epikardialen Region (206) umfasst.

7. Verfahren nach Anspruch 1, wobei das In-Beziehung-Setzen der ersten Region des Gefäßes, die in der Vielzahl von extravaskulären Bildern dargestellt ist, mit der festgelegten Rückzugslänge das Skalieren einer Lumengröße, die in mindestens einem der Vielzahl von extravaskulären Bildern dargestellt ist, umfasst.

8. Verfahren nach Anspruch 1, wobei die Vielzahl von extravaskulären Bildern von einer Vielzahl von Positionen relativ zu dem Gefäß aufgenommen werden.

9. Verfahren nach Anspruch 8, das weiter das Analysieren, durch den einen oder mehrere Prozessoren, der Vielzahl von extravaskulären Bildern umfasst, um eine dreidimensionale Ausrichtung des einen oder mehrerer Objekte relativ zu dem Gefäß zu bestimmen.

10. Verfahren nach Anspruch 9, wobei das eine oder mehrere Objekte eine Verzweigung von dem Gefäß umfassen und wobei die dreidimensionale Ausrichtung der Verzweigung eine Position und einen Abzweigewinkel der Verzweigung relativ zu dem Gefäß umfasst.

11. Verfahren nach Anspruch 10, das weiter das Berechnen, durch den einen oder mehrere Prozessoren, einer virtuellen Flussreserve, VFR, des Gefäßes auf der Basis der festgestellten Position und des Abzweigewinkels der Abzweigung umfasst.

12. System zur Bestimmung von Attributen eines Blutgefäßes, wobei das System angeordnet ist, um die Verfahrensschritte nach einem der vorhergehenden Ansprüche auszuführen.

13. Computerprogramm, das Befehle umfasst, die, wenn das Programm von einem Computer ausgeführt wird, den Computer veranlassen, die Schritte nach einem der Ansprüche 1 bis 11 auszuführen.

## Revendications

1. Procédé mis en œuvre par ordinateur pour identifier des attributs d'un vaisseau sanguin (200, 302) en utilisant une pluralité d'images extravasculaires d'un vaisseau prises pendant un retrait d'une sonde d'imagerie intravasculaire présentant une longueur de retrait définie le long d'une première région du vaisseau qui a été imagée par la sonde d'imagerie intravasculaire pendant le retrait, le procédé comprenant les étapes consistant à :
détecter, par l'intermédiaire des un ou plusieurs processeurs, des emplacements d'un ou plusieurs marqueurs (312, 312') dans la pluralité d'images extravasculaires ;
comparer, par l'intermédiaire des un ou plusieurs processeurs, une longueur apparente de la première région (208) du vaisseau (202) avec la longueur de retrait définie, la longueur apparente étant représentée dans la pluralité d'images extravasculaires par des emplacements des un ou plusieurs marqueurs (312, 312') dans la pluralité d'images extravasculaires ; et
déterminer, par l'intermédiaire des un ou plusieurs processeurs, une taille d'une seconde région (204, 206) du vaisseau représentée dans la pluralité d'images extravasculaires mais non imagée par la sonde intravasculaire pendant le retrait, en utilisant un résultat de la comparaison de la longueur apparente de la première région du vaisseau avec la longueur de retrait définie.

2. Procédé selon la revendication 1, dans lequel la taille de la seconde région (204, 206) du vaisseau inclut au moins une parmi une longueur de la seconde région, un diamètre de section transversale à l'intérieur de la seconde région, et une aire de section transversale à l'intérieur de la seconde région.

3. Procédé selon la revendication 1, comprenant en outre le calcul, par l'intermédiaire des un ou plusieurs processeurs, d'une réserve d'écoulement virtuelle, VFR, du vaisseau sur la base d'une pluralité d'images capturées par la sonde d'imagerie intravasculaire et sur la base de la taille déterminée de la seconde région du vaisseau.

4. Procédé selon la revendication 3, dans lequel la VFR du vaisseau est calculée sur la base d'une distance entre une ligne centrale de vaisseau (604) et une limite du vaisseau, la seconde région du vaisseau étant identifiée dans au moins une de la pluralité d'images extravasculaires.

5. Procédé selon la revendication 1, dans lequel la seconde région du vaisseau (202) inclut au moins une parmi une région épicardique distale (204) et une région épicardique proximale (206) du vaisseau.

6. Procédé selon la revendication 5, dans lequel la détermination d'une taille d'une seconde région du vaisseau inclut une détermination d'une longueur de la région épicardique distale (204) et d'une longueur de la région épicardique proximale (206).

7. Procédé selon la revendication 1, dans lequel la corrélation de la première région du vaisseau représentée dans la pluralité d'images extravasculaires avec la longueur de retrait définie inclut une mise à l'échelle d'une taille de lumière représentée dans au moins une de la pluralité des images extravasculaires.

8. Procédé selon la revendication 1, dans lequel la pluralité d'images extravasculaires sont prises à partir d'une pluralité d'emplacements par rapport au vaisseau.

9. Procédé selon la revendication 8, comprenant en outre l'analyse, par l'intermédiaire des un ou plusieurs processeurs, de la pluralité d'images extravasculaires de manière à identifier une orientation tridimensionnelle d'un ou plusieurs objets par rapport au vaisseau.

10. Procédé selon la revendication 9, dans lequel les un ou plusieurs objets comprennent une branche à partir du vaisseau et dans lequel l'orientation tridimensionnelle de la branche inclut une position et un angle de dégagement de la branche par rapport au vaisseau.

11. Procédé selon la revendication 10, comprenant en outre le calcul, par l'intermédiaire des un ou plusieurs processeurs, d'une réserve d'écoulement virtuelle, VFR, du vaisseau sur la base de la position identifiée et de l'angle de dégagement de la branche.

12. Système pour identifier des attributs d'un vaisseau sanguin, le système étant agencé pour exécuter les étapes de procédé selon l'une quelconque des revendications précédentes.

13. Programme informatique comprenant des instructions qui, lorsque le programme est exécuté par un ordinateur, amènent l'ordinateur à exécuter les étapes selon l'une quelconque des revendications 1 à 11.
